# DEMANDE DE BREVET EUROPEEN

(11) **EP 1 884 249 A1**
(43) Date de publication de la demande: **06.02.2008**
(21) Numéro de dépôt: 06300850.2
(22) Date de dépôt: 01.08.2006
(51) Int. Cl.: A61L 2/14

(54) **Procédé de traitement de bouteilles plastiques par plasma froid et dispositif permettant sa mise en oeuvre**

(71) Demandeur: L'AIR LIQUIDE, Société Anonyme pour l'Etude et l'Exploitation des Procédés Georges Claude, 75007 Paris (FR)
(72) Inventeur: Rostaing, Jean-Christophe, 78000 Versailles (FR)
(74) Mandataire: Mellul-Bendelac, Sylvie Lisette

(57) **Abrégé**

La présente invention porte sur un procédé de traitement de bouteilles comprenant une opération de stérilisation par plasma froid à partir de gaz non germicides et/ou une opération de dépôt de couche barrière de diffusion par plasma froid, ledit procédé étant caractérisé par le fait que ledit plasma froid délivre une énergie non thermique maximale sur l'ensemble de la surface interne de la bouteille, ledit plasma froid étant généré par un applicateur de champ à ondes de surface alimenté par un générateur de micro-ondes ou par un système à anode creuse conformée à la bouteille et alimentée en courant continu pulsé et/ou en tension radio-fréquence, la paroi de ladite bouteille constituant le diélectrique à la surface duquel l'intensité du plasma est maximale. Elle porte également sur les dispositifs permettant la mise en oeuvre Du dispositif.

## Description

L'invention a pour objet un procédé de traitement en continu de bouteilles par plasma froid, en particulier de bouteilles plastiques destinées à contenir des liquides, notamment alimentaires ou pharmaceutiques. Elle a également pour objet des dispositifs permettant de mettre en oeuvre ce procédé.

Rappelons que par plasma « froid », on entend un plasma où seuls les électrons libres dans le gaz sont portés à un niveau d'énergie moyen élevé par l'excitation électrique, alors que les molécules et atomes du gaz conservent une énergie moyenne thermique correspondant quasiment à l'ambiante.

Le conditionnement aseptique en bouteilles plastiques de liquides est un secteur en expansion de l'activité d'emballage alimentaire. Il permet d'augmenter la durée de vie et/ou d'améliorer la sécurité microbiologique. Il est destiné :
- d'une part aux eaux minérales dont on craint la contamination par des germes pathogènes, et
- d'autre part aux produits stérilisés à ultra-haute température (UHT) à longue conservation, pour ne pas réintroduire de germes susceptibles de rendre les produits impropres à la consommation (lait, soupes, jus de fruits).

En outre, pour le conditionnement de certains de ces produits, il existe un besoin d'augmenter l'imperméabilité de la bouteille afin de ralentir les transferts d'espèces gazeuses ou volatiles vers et depuis l'extérieur, notamment pour éviter la perte de CO₂ dans les boissons gazeuses et la bière, la pénétration d'oxygène et/ou la migration des arômes.

Ces opérations de stérilisation et éventuellement d'imperméabilisation doivent être intégrées dans la chaîne d'embouteillage qui va du moulage de la bouteille jusqu'au remplissage des bouteilles.

Ainsi, dans une unité d'embouteillage on effectue successivement :
- le moulage des bouteilles par extrusion-soufflage de préformes ;
- la réalisation optionnelle d'une barrière de diffusion, lorsque celle-ci ne résulte pas directement d'un multicouche incluant un polymère barrière ;
- la stérilisation de la bouteille finie ;
- le remplissage avec le liquide préalablement aseptisé ;
- et le bouchage après stérilisation du bouchon lui-même.

Dans cette industrie, l'augmentation des cadences et la réduction des coûts sont une préoccupation essentielle. La succession d'opérations précitées relève, pour chacune d'entre elles, d'une technologie particulière sur une machine dédiée et implique des transferts entre plusieurs stations de la chaîne. On cherche donc à réduire la durée de chaque étape, en adaptant ou en changeant la technologie, et à minimiser le nombre de transferts entre différentes stations de la chaîne.

Classiquement, sur les chaînes d'embouteillage existantes, la stérilisation s'effectue au moyen de liquides germicides chimiques oxydants comme le peroxyde d'hydrogène, l'acide peracétique, l'eau ozonée, etc... La bouteille est trempée ou aspergée intérieurement, éventuellement chauffée, rincée et séchée avant d'être remplie. Le procédé est efficace, mais il génère des effluents liquides dont le coût de retraitement s'ajoute à celui du procédé. De plus, d'une manière générale, la gestion de circuits d'eau crée toujours un risque de développement d'une contamination microbienne inopinée et incontrôlable, que les industriels du secteur souhaiteraient éliminer.

Pour les autres types d'emballages de produits alimentaires liquides, tels que les briques en multicouches carton/alu/polymère, la stérilisation est réalisée par des rayonnements ultra-violets, notamment en mode pulsé, associés ou non à l'application d'un liquide germicide oxydant. Dans le cas de l'association des rayonnements ultra-violets et d'un liquide germicide, il existe un effet de synergie et la stérilisation peut être très rapide. Cette méthode, bien adaptée pour traiter les surfaces internes d'aluminium de ces briques est cependant un traitement trop agressif pour être appliqué à des bouteilles. En outre, l'utilisation de lampes UV présente l'inconvénient que leur rayonnement est directionnel, émis dans un angle solide bien défini et limité. Il est donc soumis, avant d'atteindre les germes à inactiver, à des effets d'ombrage du fait de la géométrie du récipient traité. Cette méthode n'est donc pas appropriée à la géométrie des bouteilles.

Des stérilisations par plasma ont été envisagées. Ainsi, dans le document EP-1 068 032, on évoque la possibilité de réduire la contamination microbienne sur la paroi interne de la bouteille au moyen d'un plasma micro-ondes d'oxygène excité in-situ (sans autre précision). Cependant, il est dit que l'efficacité est insuffisante pour que l'on puisse se passer d'une association avec une étape liquide dans un second temps. Aucun mécanisme d'action du plasma n'est évoqué.

D'autres auteurs (voir par exemple les documents US-6627163, US-5904866, US2005/0019209) présentent des solutions où les bouteilles sont placées dans un « bain » de plasma ou de post-décharge émis par une source dont la géométrie est décorrélée de celle des bouteilles, de sorte que le plasma au niveau de la paroi de ces dernières est inhomogène. Ces arrangements n'ont pas d'intérêt pratique car, dans ce cas, le maximum de densité des espèces actives générées au sein du plasma et leur énergie, n'est pas atteint au niveau de la paroi de la ou les bouteilles, ou en tout cas pas en tous les points de cette surface, et l'efficacité sera à certains endroits très insuffisante.

En ce qui concerne l'imperméabilisation des bouteilles, différentes solutions sont proposées.

Dans la présente invention, on utilisera indifféremment les termes « imperméabilisation » ou « dépôt d'une couche barrière de diffusion » pour désigner l'opération consistant à déposer sur une surface de la bouteille une couche permettant de limiter la diffusion de molécules gazeuses de l'extérieur de la bouteille vers l'intérieur de celle-ci et de l'intérieur de la bouteille vers l'extérieur.

Les solutions basées sur une coextrusion de multicouches présentent des risques de délamination, et sont coûteuses. Les enductions de résine sont peu efficaces et posent des problèmes de recyclage. Dans les deux cas, le polymère barrière reste au contact du liquide et peut interagir avec lui, générant ainsi des transferts de contaminants chimiques.

Une autre solution a été envisagée par la société SIDEL (procédé commercial connu sous le nom de « ACTIS ») et consiste à déposer par plasma CVD (appelé encore PECVD) une barrière de diffusion de quelques centaines de nanomètres à l'intérieur de bouteilles déjà formées.
Pour cela, une vapeur de monomère est excitée par un plasma micro-ondes entretenu à l'intérieur de la bouteille. Le plasma dissocie les molécules de monomère source pour donner des radicaux d'énergie interne élevée qui sont des précurseurs très actifs de dépôt solide. Ces radicaux précurseurs, lorsqu'ils vont arriver sur la surface de la bouteille, vont se condenser et s'incorporer dans la phase solide du film mince en cours de croissance.

La source de plasma est, en fait, une structure de cavité résonnante alimentée via un guide d'ondes. La bouteille est placée dans une enceinte diélectrique d'un diamètre légèrement supérieur, elle-même disposée dans la structure conductrice de la cavité résonnante. Le procédé de dépôt nécessite un vide de l'ordre de 0,1 mbar à l'intérieur de la bouteille, impliquant une installation de pompage de taille suffisante. L'enceinte entourant la bouteille est également pompée, mais à un vide moins élevé, pour éviter la contraction et l'écrasement de la bouteille, et aussi pour empêcher l'allumage parasite d'un second plasma à l'extérieur.

Compte tenu du mode d'excitation du plasma, par cavité résonnante, l'application est limitée aux bouteilles de petites dimensions. En effet, la distribution d'intensité du champ électrique dans la cavité, et donc la répartition de densité du plasma, est imposée par les modes électromagnétiques propres de la cavité. Il n'existe pas de tel mode pour lequel le champ soit suffisamment homogène axialement pour traiter des bouteilles d'une contenance supérieure à, environ, 0,60 litres.

Dans le cas du dispositif plasma micro-ondes « SIDEL ACTIS », de façon inhérente à l'excitation par cavité résonnante, le champ micro-ondes ne possède pas son maximum d'intensité au voisinage de la surface de la bouteille. En conséquence la création d'espèces de plus haute énergie interne n'est pas particulièrement favorisée dans cette zone.

Il n'est pas possible non plus, dans cet agencement, d'amplifier et de contrôler le bombardement de la surface interne de la bouteille par des ions du plasma. La bouteille est en matériau diélectrique et il n'y a pas de moyen évident de la charger négativement de manière répartie et modulable. On ne peut pas, par exemple, appliquer uniformément une polarisation radiofréquence à cette surface au moyen d'une électrode conductrice entourant la bouteille car alors, les micro-ondes ne pourraient plus traverser la paroi de la bouteille pour entretenir le plasma à l'intérieur de celle-ci.

Une autre solution pourrait être d'injecter des électrons rapides produits par un canon à électrons vers la surface, comme cela a été proposé par certains auteurs, mais cette alternative n'est ni simple ni bon marché et sa praticabilité à l'intérieur de la bouteille reste hypothétique.

Il existe donc un réel besoin en un procédé permettant de stériliser et/ou de déposer des couches d'imperméabilisation, ledit procédé étant destiné à être intégré dans un procédé classique d'embouteillage, et ne générant pas d'effluents aqueux, n'utilisant pas de composés chimiques germicides et mis en oeuvre avec un nombre limité d'étapes de transferts.

La présente invention permet de répondre à ce besoin grâce à la réalisation d'une stérilisation uniquement par plasma froid à partir de gaz non germicides et à la réalisation de l'imperméabilisation à l'aide d'un plasma froid, les deux étapes pouvant être réalisées dans un unique dispositif.

Dans la présente invention, on désigne par « gaz non germicides », les gaz qui ne présentent pas d'activité germicide dans les conditions normales, c'est-à-dire en l'absence de plasma.

Ainsi la présente invention porte sur un procédé de traitement de bouteilles comprenant une opération de stérilisation par plasma froid à partir de gaz non germicides et/ou une opération de réalisation de dépôt de couche barrière de diffusion par plasma froid, ledit procédé étant caractérisé par le fait que ledit plasma délivre une énergie non thermique maximale sur l'ensemble de la surface interne de la bouteille.

L'énergie peut être de nature interne aux espèces et due à l'excitation de niveaux quantifiés électroniques et vibrationnels ou est générée par l'impact cinétique des ions accélérés par un champ électrique appliqué et bombardant la surface.

Le plasma généré présente également une densité électronique élevée sur l'ensemble de la surface interne de la bouteille. Dans un plasma, le taux de création de toutes les espèces actives croît avec la densité électronique, et il lui est même généralement proportionnel. Cela va être vrai pour les espèces excitées émettrices d'UV et les radicaux oxydants ou réducteurs impliqués dans la stérilisation, cela va être également le cas pour les radicaux précurseurs de dépôt PECVD. Cette forte densité d'espèces actives, jointe au fait que l'énergie non thermique moyenne soit maximale à la surface interne de la bouteille permet à la fois de réduire le temps de stérilisation et le temps de dépôt de la barrière de diffusion.

Conformément à l'invention, le plasma froid est généré par un applicateur de champ à ondes de surface alimenté par un générateur de micro-ondes ou par un système à cathode creuse conformée à la bouteille et alimentée en courant continu pulsé et/ou en tension radio-fréquence, la paroi de ladite bouteille constituant le diélectrique à la surface duquel l'énergie non thermique moyenne des espèces est maximale.

Selon un premier mode de réalisation, le plasma est généré par des micro-ondes transmises par un applicateur de champ à onde de surface appelé également lanceur d'onde de surface.
Cet applicateur se trouve sous la forme d'une structure conductrice traversée localement par l'enceinte diélectrique, constituée par la bouteille, le long de laquelle on lance l'onde de surface pour y entretenir un plasma.
De tels lanceurs d'onde de surface sont connus. Une description détaillée du concept est donnée dans le chap. 5 de l'ouvrage Microwave Excited Plasmas, Eds. M. Moisan and J. Pelletier, Elsevier, Amsterdam, 1992. A titre d'exemples pouvant être utilisés pour l'invention, on peut citer le robox et le surfatron qui sont alimentés en puissance à partir d'une ligne coaxiale, et le surfaguide et le surfatron-guide qui sont alimentés en puissance par un guide d'ondes rectangulaire creux.

Le choix du lanceur d'ondes de surface dépendra du type de propriétés recherchées. Ainsi, l'alimentation en micro-ondes par un guide d'ondes permet de faire passer des puissances supérieures, mais la ligne coaxiale peut prendre la forme d'un câble souple ou semi-rigide, ce qui peut faciliter la mise en place du dispositif sur la bouteille à chaque cycle de traitement pour une cadence élevée.

Une caractéristique essentielle de la méthode d'excitation d'un plasma par une onde de surface, est que cette dernière est supportée par le plasma lui-même. La propagation de l'onde de surface est guidée à l'interface entre le plasma conducteur et la surface du diélectrique constituée par la bouteille, grâce à cette discontinuité de conductivité. L'onde possède son intensité maximale au niveau de cette interface, c'est-à-dire à la surface même de la bouteille. Ainsi une densité maximale d'énergie est déposée dans le plasma à ce niveau, se retrouvant notamment sous forme d'énergie non-thermique des particules. Dans le cas présent il s'agit essentiellement d'énergie interne des espèces sous forme d'excitation des niveaux électroniques et vibrationnels quantifiés, et non d'énergie cinétique des ions. A partir du lanceur d'onde de surface, l'onde se propage le long de la paroi diélectrique parallèlement à l'axe de symétrie de la bouteille, en s'amortissant graduellement au fur et à mesure qu'elle cède sa puissance qui est absorbée pour entretenir le plasma.

L'onde s'étend jusqu'au point où il n'y a plus assez de puissance pour que le plasma puisse exister et continuer de supporter la propagation. Lorsqu'on augmente la puissance des micro-ondes, l'onde peut se propager et entretenir le plasma sur une distance plus longue, le plasma s'étend alors de plus en plus loin, ce qui permet d'adapter l'étendue de la zone de traitement, sans rien modifier à la structure de l'applicateur de champ.

Les applicateurs de champs à onde de surface fonctionnent généralement de manière symétrique, c'est-à-dire que deux ondes sensiblement identiques peuvent être lancées de part et d'autre dans des directions opposées. L'intensité de chaque onde, et par conséquent la densité du plasma, diminuent cependant progressivement (en fait, quasi linéairement) à partir de l'applicateur du fait de l'absorption de la puissance au fur et à mesure de la propagation de l'onde de surface.

Ainsi, selon un mode de réalisation particulier, l'applicateur de champ à onde de surface est placé dans la zone médiane de la bouteille, à partir le laquelle deux ondes opposées vont se propager respectivement vers le col et vers le fond. Compte tenu du resserrement du col de la bouteille, l'onde va s'amortir beaucoup moins rapidement lors de sa propagation dans le sens du col que dans le sens du fond du fait du resserrement du diamètre (moins de volume de plasma à entretenir donc moins de puissance utilisée). De ce fait, l'applicateur pourra être positionné plus près du fond que du col. Il faudra également veiller à la forme prise par l'onde lorsqu'elle épouse la courbure du fond de la bouteille, et en particulier adapter la puissance et/ou la distance de l'applicateur au fond de manière à ce qu'il ne se construise pas de phénomène d'interférence trop prononcé nuisible à l'homogénéité et au contrôle du procédé. Ces adaptations constituent des opérations d'optimisation pour l'homme du métier.

Selon un second mode de réalisation du procédé de l'invention, le plasma est généré par un dispositif plasma à cathode creuse qui permet, tout comme dans le cas de l'excitation par des micro-ondes, d'entretenir un plasma de haute densité électronique, c'est-à-dire de grande efficacité pour créer des espèces actives comme des radicaux.

Le principe de la cathode creuse est totalement différent de celui de l'onde de surface. Aux fréquences intermédiaires entre le continu et les radiofréquences, un plasma est généralement excité entre deux électrodes conductrices reliées aux pôles d'un générateur (structure diode). A ces fréquences, le taux de création continu de paires électrons-ions, par collisions inélastiques des particules chargées déjà existantes sur les molécules du gaz, est nettement moins élevé qu'en micro-ondes (en alternatif, la densité du plasma croît approximativement comme la racine carrée de la fréquence).

Dans la structure diode, il n'y a pas de confinement des espèces chargées susceptibles d'augmenter leur durée de vie en retardant leurs pertes. En particulier, l'anode collecte les électrons qui se recombinent et disparaissent sur sa surface, qu'il s'agisse des électrons créés par des collisions inélastiques en volume, ou de ceux générés dans le « régime gamma » à la suite du bombardement de la cathode par des ions énergétiques.

L'agencement dit « de cathode creuse » permet de conserver plus longtemps les électrons énergétiques dans le plasma, et d'augmenter l'efficacité d'ionisation et la densité moyenne d'espèces chargées. Le concept est basé sur une géométrie où la cathode détermine une cavité aux parois conductrices, qui entoure le plasma dans pratiquement toutes les directions, excepté une ou plusieurs petites ouvertures par lesquels les lignes de champ peuvent retourner à une anode externe.

Pour obtenir l'effet souhaité sur la densité du plasma, les conditions doivent être ajustées de telle manière que le libre parcours moyen des électrons soit légèrement inférieur au diamètre de la cathode creuse. Les électrons repoussés par la cathode ont ainsi une forte probabilité d'atteindre le coeur du plasma, puis d'y induire sur les molécules initialement neutres des collisions inélastiques créant de nouvelles paires électron-ion, processus qui finalement augmentent la densité de charge. Cette dernière est typiquement plus importante d'un ordre de grandeur par rapport à un système diode, c'est-à-dire comparable à celle que l'on peut réaliser dans un système micro-ondes.

Le libre parcours moyen est principalement une fonction de la pression du gaz, qui doit donc être choisie de manière appropriée selon le diamètre de la cathode creuse.

Selon un mode de réalisation particulièrement avantageux, la cathode creuse est conformée à la forme de la bouteille qui est placée à l'intérieur et le plasma est entretenu de manière permanente en appliquant une polarisation négative continue pulsée, ou une polarisation radiofréquence, ou une combinaison des deux. En revanche, la bouteille étant en matériau diélectrique, on ne peut pas utiliser une polarisation négative continue permanente. En effet, dans ce cas, la surface interne collectant des ions positifs du plasma prendrait une charge positive qui augmenterait progressivement. Le champ électrique créé par cette charge s'opposerait au champ excitateur externe accélérant les électrons et, finalement, provoquerait l'extinction du plasma.

De façon avantageuse, une réplique du moule d'extrusion ou le moule d'extrusion lui-même peut être utilisé pour réaliser cette cathode creuse. L'anode externe peut être disposée dans le prolongement du col de la bouteille, sur la canalisation servant à l'alimentation en gaz et au pompage, avec une isolation électrique intermédiaire.

Afin d'éviter l'allumage du plasma dans l'espace compris entre la bouteille et la cathode creuse et également empêcher la déformation et l'écrasement de la bouteille, un vide moins important que dans la bouteille est établi dans l'espace compris entre la cathode creuse et la bouteille.

Lorsque l'espace entre la cathode et l'empreinte du moule est très étroit, cet espace peut même être maintenu à la pression atmosphérique sans que les problèmes mentionnés ci-dessus n'apparaissent.

La cathode creuse peut être alimentée en courant continu pulsé avec une amplitude, une durée d'impulsion et un taux de répétition ajustable. Le choix de ces paramètres permet de contrôler avec un certain degré d'indépendance la densité du plasma et la valeur moyenne du potentiel de polarisation en surface, donc le bombardement ionique de la paroi interne de la bouteille. C'est ce dernier bombardement ionique qui représente principalement dans ce cas l'apport d'énergie non thermique sur la surface interne de la bouteille. La modulation de l'alimentation pulsée a normalement moins d'intérêt ici pour améliorer l'uniformité de dépôt en atténuant l'effet de déplétion de la phase gazeuse en précurseurs (en permettant leur réapprovisionnement entre chaque cycle). En effet, les dimensions internes de la bouteille ne sont pas si étroites que cet effet doive être important.

En variante ou en complément, la cathode peut être polarisée par l'application d'une tension radiofréquence. Dans ce cas, l'effet d'autopolarisation existe de la même manière que dans un système d'électrodes diode classique. Comme, d'une manière générale, les électrons sont plus mobiles dans le plasma que les ions, la charge négative collectée par la paroi de la bouteille au contact de la cathode creuse, lors d'une alternance positive, est plus grande en valeur absolue que la charge positive collectée pendant une alternance négative. Le diélectrique prend alors une charge permanente négative et un potentiel continu de même signe, induisant un bombardement ionique continu de la surface interne de la bouteille. On peut grâce à cette autopolarisation, dont l'amplitude est modulable en jouant sur les paramètres de l'excitation radiofréquence, ajuster avec plus de liberté l'énergie cinétique des ions accélérés arrivant sur la surface interne de la bouteille, c'est-à-dire l'énergie non-thermique déposée sur cette dernière. Dans le cas d'une excitation radiofréquence, le dispositif comprend un blindage radiofréquence à la masse autour de la cathode creuse avec de l'air, ou un diélectrique solide, entre les deux conducteurs.

Conformément à l'invention, la stérilisation et le dépôt de la couche barrière de diffusion sont réalisées dans le même dispositif générateur de plasma. Bien entendu, suivant l'étape souhaitée les conditions de génération du plasma et les gaz mis en oeuvre seront différents.

Ainsi, le plasma mis en oeuvre pour la stérilisation comprend des gaz choisis dans le groupe comprenant N₂, O₂, N₂O, H₂, H₂O (vapeur d'eau), Ar, He, Kr, Xe ou leurs mélanges.

De façon avantageuse, on utilise un mélange N₂/O₂. De préférence le mélange N₂/O₂ est un mélange plus riche en oxygène que ceux utilisés pour la stérilisation médicale, par exemple dans un ratio molaire N₂/O₂ de 95/5 à 80/20.

La bouteille est mise sous un vide de l'ordre de 0,1 à 10 mbar et la stérilisation est conduite dans un temps aussi court que celui utilisé dans les méthodes classiques de stérilisation par utilisation de solutions aqueuses germicides. La durée de l'étape de stérilisation est de 5 à 0,05 secondes, de préférence de 2 à 0,1 seconde et plus préférentiellement encore de 1 à 0,5 seconde.

L'homme du métier est en mesure de régler les conditions du plasma de telle sorte que l'intensité du plasma soit suffisante pour stériliser sans entraîner la dégradation de la structure du polymère et la génération d'espèces chimiques incompatibles avec un usage alimentaire ni de surchauffe du polymère.

Les mécanismes d'inactivation de micro-organismes par ces plasmas sont bien expliqués et les espèces actives impliquées sont identifiées. Les germes sont tués par trois types de mécanismes : les rayonnements ultra-violets émis par la désexcitation de certains niveaux énergétiques de molécules, ions et radicaux, les radicaux oxydants ou réducteurs atteignant le matériel génétique après avoir diffusé à travers les couches organiques périphériques et l'érosion physique ou chimique de la matière des micro-organismes.

La stérilisation par plasma selon le mode exposé ici, c'est-à-dire sans utiliser aucun produit source chimique, mais uniquement des gaz qui n'acquièrent leurs propriétés germicides que dans le plasma du fait de l'excitation électromagnétique, est un procédé entièrement sec mais aussi un procédé intrinsèquement propre. En effet les espèces actives responsables de l'inactivation des germes, radicaux oxydants et différentes autres espèces excitées, ont une existence transitoire et disparaissent rapidement lorsque le gaz sort de la zone de plasma, en se désexcitant et/ou se recombinant pour reformer les espèces du gaz initial comme O₂ et N₂, plus éventuellement une faible proportion d'oxydes d'azote. Ces derniers, sont faciles à éliminer sur un dispositif peu coûteux, par exemple un système à adsorption réactive. La durée de vie de l'adsorbant consommable est importante du fait de la faible concentration de polluants à traiter.

L'étape de stérilisation peut faire l'objet d'un contrôle in-situ en acquérant un paramètre physique indicatif de l'espèce ou des espèces reconnues comme principales responsables du processus d'inactivation. Par exemple un détecteur optique peut suivre un signal caractéristique d'un radical oxydant ou réducteur identifié, ou bien le niveau d'intensité des UV dans une certaine bande spectrale, etc...

Pour ce qui est de l'étape de dépôt d'une barrière de diffusion, on utilisera comme gaz dans le plasma, différents monomères, notamment des vecteurs de carbone comme des hydrocarbures, ou encore des composés du silicium en fonction de la nature du dépôt envisagé.

En effet, la couche barrière de diffusion peut être de toute composition appropriée, notamment un alliage amorphe de silicium, comme un oxyde stoechiométrique ou non, un nitrure, un oxynitrure, etc... ou un composé solide de carbone, comme du carbone amorphe hydrogéné sous ses différentes formes. La couche barrière peut présenter une structure multicouches ou un gradient de propriétés selon son épaisseur. Par exemple, on peut déposer une couche à caractère plus polymérique et organique au voisinage de l'interface, pour favoriser l'adhésion et la tenue thermomécanique, et une couche plus dense, dure et inorganique au niveau de la surface externe. Le substrat peut être préparé avant dépôt pour une meilleure adhésion, par tout type de prétraitement plasma à base d'argon, d'azote, d'oxygène, etc...

Les caractéristiques d'une couche déposée par PECVD dépendent beaucoup des paramètres physicochimiques du plasma, c'est-à-dire indirectement du dispositif qui sert à produire ce dernier. D'une manière générale, pour un dépôt réalisé à froid, la « qualité » des couches déposées est meilleure lorsque l'énergie moyenne (non-thermique) apportée par les espèces du plasma est élevée.

Par « qualité » d'une couche mince déposée, on entend une structure dense et une bonne « cohésion » de la matière solide. Ceci peut être vrai à l'échelle atomique avec une bonne connectivité du réseau d'atomes, un minimum de liaisons chimiques pendantes, de lacunes, et de nano-pores, aussi bien qu'à l'échelle de l'épaisseur de la couche (quelques centaines de nm à quelques microns) avec absence de structures granulaires ou colonnaires qui sont bien visibles au microscope électronique. On conçoit qu'une couche mince formée d'un matériau satisfaisant à ces critères, soit plus résistante mécaniquement et qu'elle constitue une barrière plus efficace aux agents physiques et chimiques.

Pour obtenir une telle qualité du matériau, il faut que les atomes arrivant sur la surface de la couche mince en croissance puissent s'incorporer en phase solide avec le bon arrangement. Pour cela, de l'énergie doit être apportée sur la surface pour assister la migration des atomes vers les configurations les plus stables et les plus régulières. Cette énergie est également nécessaire pour dissocier d'éventuels amas d'atomes qui arriveraient sur la surface, après s'être formés dans la phase gazeuse par interaction prématurée entre les radicaux précurseurs du dépôt. Faute de quoi, l'arrangement (généralement impropre) de ces atomes au sein des amas, serait conservé dans la couche mince solide. Selon la chimie de précurseurs et le type de matériau que l'on veut déposer par PECVD, la formation prématurée d'amas d'atome dans la phase gazeuse est plus ou moins probable, et ces amas vont être plus pou moins faciles à réarranger par la suite en surface. Ainsi, un procédé PECVD conduit dans des conditions où l'énergie non thermique moyenne déposée sur la surface sur laquelle on fait croître le film mince, est maximale, offre la possibilité d'obtenir une couche barrière de qualité optimale pour une plus large gamme de précurseurs gazeux et donc de compositions du matériau déposé. Un tel procédé PECVD peut également dans certains cas être conduit à une pression plus élevée qu'usuellement (cette dernière favorisant les réactions prématurée en phase gazeuse) sans perte sensible de qualité du matériau.

Selon un mode de réalisation particulier, le procédé de l'invention, comprend une première étape de stérilisation puis une seconde étape de dépôt de barrière de diffusion et éventuellement une troisième étape de « finition » de la stérilisation.
Ce mode de réalisation est particulièrement avantageux lorsque la stérilisation est conduite dans des conditions « dures » de plasma permettant une stérilisation très rapide. Ainsi, même si ces conditions conduisent à une légère altération de la structure de surface, le matériau polymère une fois revêtu de sa barrière inorganique, devrait recouvrer ses propriétés de compatibilité alimentaire. Par ailleurs, le plasma de dépôt peut lui-même contenir des espèces stérilisantes, notamment dans le cas d'un matériau SiOₓ nécessitant un gaz précurseur oxydant, et le dépôt PECVD est en principe un procédé bactériologiquement « propre ».

Une étape supplémentaire de « finition » de la stérilisation peut être envisagée, même si celle-ci n'est pas préférée du fait qu'elle serait pénalisante en termes de temps.

Selon un autre mode de réalisation particulier, le procédé de l'invention comprend une première étape de dépôt PECVD, éventuellement avec l'application d'un traitement UV, et une seconde étape de stérilisation.

Dans le cas où l'on réalise la stérilisation après le dépôt, la barrière de diffusion étant faite d'un matériau inorganique, sera beaucoup plus résistante à l'action du plasma oxydant que le polymère nu. Il faut toutefois prendre garde à l'action de photons UV, à travers la couche barrière, sur l'interface de cette dernière avec le substrat de polymère. On sait d'expérience que cela peut être un facteur de décohésion suite à la rupture de liaisons chimiques à cet interface. Pour éviter ce risque, on peut donner, s'il y a lieu, à tout ou partie de l'épaisseur de la couche déposée, des propriétés de barrière UV. Pour cela il suffit, par exemple, de moduler la composition d'une couche de SiOₓ, de manière à ajuster le seuil d'absorption à la limite spectrale entre le visible et l'UV. La transition d'absorption n'est pas abrupte mais, même si une fraction du spectre bleu/violet est absorbée, l'épaisseur de la barrière sera généralement trop faible pour qu'une coloration jaunâtre soit perceptible.

Le procédé de l'invention est intégré dans le procédé global d'embouteillage et conduit immédiatement après l'extrusion, éventuellement après refroidissement de la bouteille.

L'étape de refroidissement sera d'autant plus nécessaire que le dépôt PECVD sera effectué avant la stérilisation. En effet, même si la température favorise la qualité du dépôt, après refroidissement les contraintes thermiques différentielles entre le substrat polymérique et la couche barrière inorganique, peuvent être excessives et provoquer un décollement de la couche.

L'invention porte également sur les dispositifs générateurs de plasma froid permettant la mise en oeuvre du procédé de l'invention.

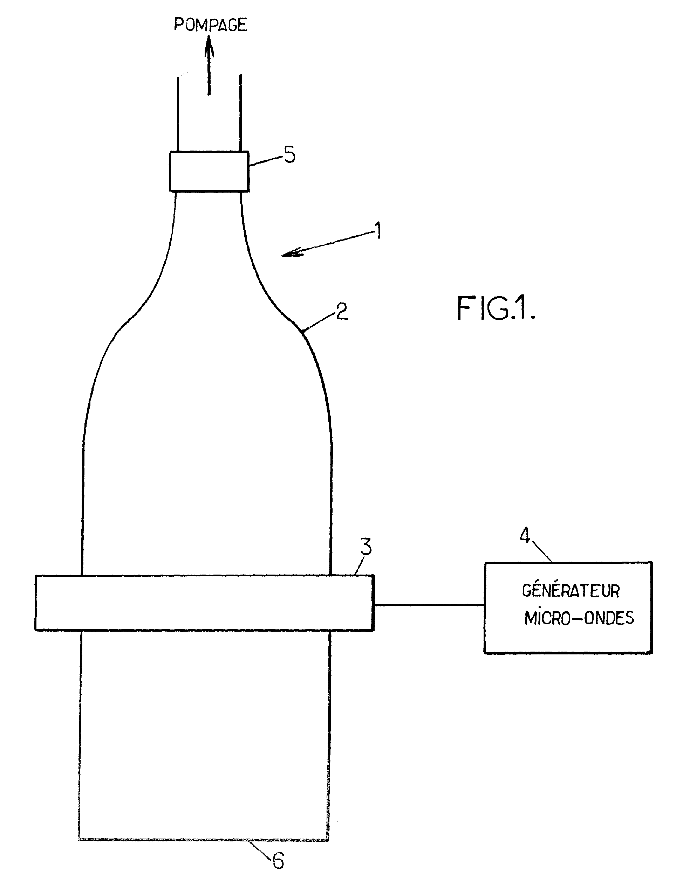

Ainsi, selon un premier mode de réalisation le dispositif de l'invention est un lanceur d'ondes de surface de forme annulaire qui est destiné à être placé autour de la bouteille à traiter. Il est de préférence placé au niveau de la partie médiane de la bouteille, préférentiellement légèrement plus proche du fond de la bouteille que de son goulot. Un tel dispositif est schématisé sur la figure 1 .

Sur la figure 1, on a représenté de façon schématique un dispositif 1 de génération de plasma froid du type lanceur d'ondes de surface. La bouteille à traiter 2 est placée à l'intérieur d'un applicateur annulaire 3 alimenté par un générateur de micro-ondes 4. Un système de pompage (non représenté) permettant de régler le vide à l'intérieur de la bouteille est disposé au niveau du goulot 5 de la bouteille 2.

Lorsque le système est en fonctionnement, le vide est fait dans la bouteille par le dispositif de pompage, qui permet également de faire circuler, sous la pression réduite requise, le flux gazeux qui sera nécessaire ou bien à la stérilisation ou bien au dépôt de la barrière de diffusion. Un montage approprié, connu de l'homme de métier, permet d'injecter le mélange de gaz approprié à l'intérieur de la bouteille. Le procédé peut aussi être conduit en régime statique en introduisant une quantité fixe de mélange gazeux. En effet la consommation relative des composants réactifs du mélange ne sera pas importante. Le générateur de micro-ondes est mis en fonctionnement et l'onde de surface se propage alors à la fois de l'applicateur annulaire 3 vers le fond 6 de la bouteille et de l'applicateur annulaire 3 vers le goulot 5.

Selon un second mode de réalisation, le dispositif générateur de plasma froid est de type à cathode creuse, la cathode creuse étant conformée à la forme de la bouteille et constituée de deux demi-coques permettant une ouverture et fermeture aisées et le plasma étant alimenté par une polarisation négative continue pulsée et/ou une polarisation radiofréquence.

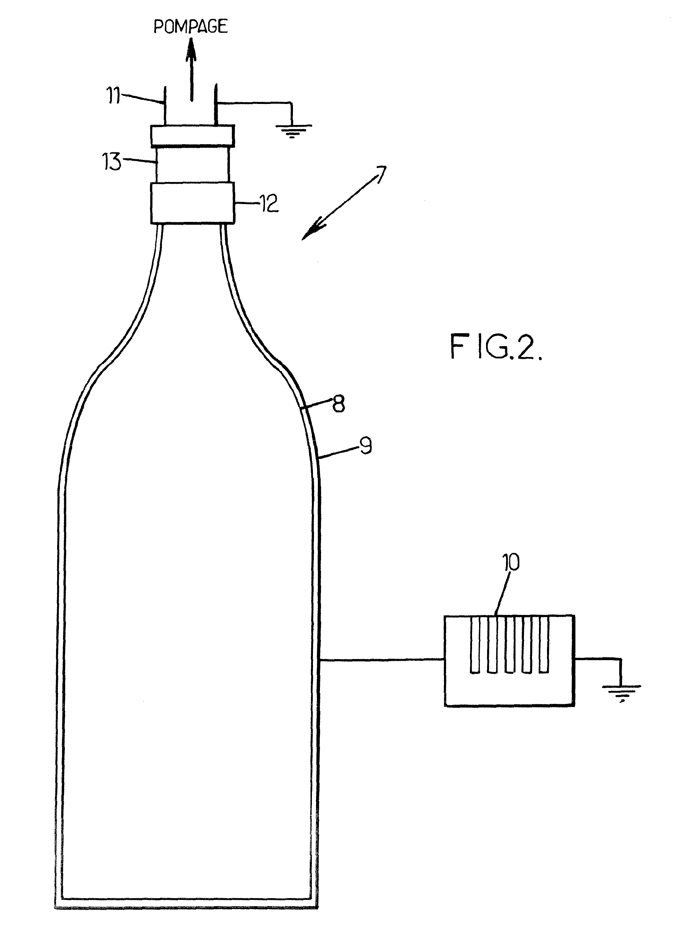

Sur la figure 2, on a représenté de façon schématique un dispositif de génération de plasma froid par cathode creuse.

Dans ce dispositif 7, la bouteille 8 est placée à l'intérieur d'une cathode creuse 9 constituée de deux demi-coques. Ladite cathode creuse 9 est conformée à la forme de la bouteille 8.

La cathode creuse 9 est alimentée en courant DC pulsé négatif par un générateur 10. L'anode 11 est disposée au niveau du goulot 12 de la bouteille. L'anode est reliée à la terre. Un élément isolant 13, disposé au niveau du goulot, sépare l'anode de la cathode. Comme dans le cas précédent, un système de pompage (non représenté) est disposé au niveau du goulot de la bouteille, ainsi qu'un dispositif d'injection des gaz pour maintenir une composition, une pression réduite et un flux donné ou nul des gaz de procédé.

L'intégration des dispositifs plasma aux infrastructures existantes sur la chaîne d'embouteillage tient compte, le cas échéant, de contraintes propres. Par exemple, dans le cas d'un dispositif à cathode creuse, la cathode étant portée à un potentiel fortement négatif par rapport à la masse, il faut donc réaliser les isolations électriques par rapport au reste de l'installation pour travailler en toute fiabilité et sécurité. Dans le cas où la cathode creuse est alimentée en radiofréquence, le blindage RF doit pouvoir être intégré en respectant l'architecture mécanique du reste de la machine. La cathode creuse est réalisée en deux demi-coquilles pour permettre le chargement et le déchargement de la bouteille.

Le dispositif peut comporter une double paroi, la cathode creuse côté interne et une anode enveloppante côté externe, avec un diélectrique entre les deux parois et des moyens pour assurer une bonne continuité électrique de chacun des conducteurs interne et externe, tout en maintenant une bonne isolation entre eux, lorsque la coquille est fermée.

Dans le cas où la cathode creuse est constituée par le moule lui-même, les liaisons mécaniques du moule au bâti devront être réalisées en matériau isolant, par exemple des pièces d'articulation en céramique.

Les moyens d'entretien du vide, d'ouverture et de fermeture rapide de l'enceinte de traitement, d'injection des gaz de procédé, de chargement, de déchargement des bouteilles et de manutention de ces dernières sont ceux classiquement utilisés dans les chaînes d'embouteillage.

L'invention est décrite de façon plus détaillée dans les exemples qui suivent qui sont donnés à titre purement illustratifs.

### EXEMPLE 1 :

L'invention peut être appliquée sur toute ligne d'embouteillage aseptique.

Une préforme en polymère est transformée en bouteille dans un dispositif d'extrusion soufflage classique. Les bouteilles, juste extrudées, sont convoyées vers une station de traitement comportant un dispositif plasma tel que schématisé sur la Figure 1.

Les moyens d'entretien du vide, d'ouverture et de fermeture rapide de l'enceinte de traitement, d'injection des gaz de procédé, de chargement et de déchargement des bouteilles et de manutention de ces dernières sont des moyens classiques utilisés sur des chaînes d'embouteillage.

Un vide de 1,0 mbar est réalisé à l'intérieur de la bouteille et un apport d'un mélange de N₂/O₂ dans un ratio molaire N₂/O₂ de 90/10 est introduit dans la bouteille. Un vide de 50 mbar autour de la paroi externe de la bouteille est créé afin d'éviter toute déformation de celle-ci.

### EXEMPLE 2 :

Une préforme en polymère est transformée en bouteille dans un dispositif d'extrusion soufflage classique. Les bouteilles, juste extrudées, sont convoyées vers une station de traitement comportant un dispositif plasma tel que schématisé sur la Figure 2, dans lequel une réplique métallique du moule d'extrusion constitue la cathode creuse.

Les moyens d'entretien du vide, d'ouverture et de fermeture rapide de l'enceinte de traitement, d'injection des gaz de procédé, de chargement et de déchargement des bouteilles et de manutention de ces dernières sont des moyens classiques utilisés sur des chaînes d'embouteillage.

Un vide de 0,2 mbar est réalisé à l'intérieur de la bouteille et un apport d'un mélange de N₂/O₂ dans un ratio molaire N₂/O₂ de 90/10 est introduit dans la bouteille.

Le niveau de stérilisation de la bouteille est suivi à l'aide d'un détecteur optique qui suit un signal caractéristique d'un radical oxydant identifié, par exemple l'oxygène atomique. Si le niveau de ce signal est resté conforme pendant un temps préalablement déterminé (environ 1 seconde), les espèces gazeuses sont alors retirées et remplacées par des espèces nécessaires à la réalisation de la barrière de diffusion.

Dans un premier temps la surface interne est prétraitée pour favoriser l'adhésion en utilisant un plasma à base d'argon ; puis, une barrière de diffusion est déposée en introduisant dans le plasma un mélange d'argon, d'oxygène, et de silane.

Lorsque l'épaisseur de la couche de diffusion est suffisante, le vide est interrompu et la bouteille est sortie du moule d'extrusion puis est refroidie avant d'être convoyée vers la station de remplissage. Simultanément, une nouvelle préforme est introduite dans le moule d'extrusion soufflage.

## Revendications

1. Procédé de traitement de bouteilles comprenant une opération de stérilisation par plasma froid à partir de gaz non germicides et/ou une opération de dépôt de couche barrière de diffusion par plasma froid, ledit procédé étant **caractérisé par le fait que** ledit plasma froid délivre une énergie non thermique maximale sur l'ensemble de la surface interne de la bouteille.

2. Procédé selon la revendication 1, **caractérisé par le fait que** les opérations de stérilisation et de dépôt de couche barrière de diffusion sont réalisées dans un seul et même dispositif.

3. Procédé selon la revendication 1 ou 2, **caractérisé par le fait que** le plasma froid est généré par un applicateur de champ à ondes de surface alimenté par un générateur de micro-ondes ou par un système à anode creuse conformée à la bouteille et alimentée en courant continu pulsé et/ou en tension radio-fréquence, la paroi de ladite bouteille constituant le diélectrique à la surface duquel l'énergie non-thermique moyenne des espèces du plasma est maximale.

4. Procédé selon l'une quelconque des revendications 1 à 3, **caractérisé par le fait que** le plasma mis en oeuvre pour la stérilisation comprend des gaz choisis dans le groupe comprenant N₂, O₂, N₂O, H₂, H₂O, Ar, He, Kr, Xe ou leurs mélanges, de préférence un mélange N₂/O₂ et plus préférentiellement encore dans un ratio molaire N₂/O₂ de 95/5 à 80/20.

5. Procédé selon l'une quelconque des revendications 1 à 4, **caractérisé par le fait que** la durée de la stérilisation est est de 5 à 0,05 secondes, de préférence de 2 à 0,1 seconde et plus préférentiellement encore de 1 à 0,5 seconde.

6. Procédé selon l'une quelconque des revendications 1 à 5, **caractérisé par le fait que** la stérilisation est conduite avec un vide de 0,1 à 100 mbar.

7. Procédé selon l'une quelconque des revendications 1 à 6, **caractérisé par le fait que** le plasma mis en oeuvre pour le dépôt de la barrière de diffusion comprend des gaz choisis dans le groupe comprenant des monomères, des vecteurs gazeux de carbone, des composés gazeux du silicium ou leurs mélanges.

8. Procédé selon l'une quelconque des revendications 1 à 7, **caractérisé par le fait que** le dépôt de la barrière de diffusion est conduit avec un vide de 0,1 à 10 mbar.

9. Procédé selon l'une quelconque des revendications 1 à 8, **caractérisé par le fait que** la stérilisation est réalisée avant le dépôt de la couche barrière de diffusion et qu'éventuellement on ajoute une étape de finissage de la stérilisation par plasma froid.

10. Procédé selon l'une quelconque des revendications 1 à 8, **caractérisé par le fait que** le dépôt de la couche barrière de diffusion est réalisé avant l'opération de stérilisation, la couche barrière de diffusion incluant éventuellement une protection contre les rayonnements ultraviolets.

11. Dispositif de génération d'un plasma froid de type lanceur d'ondes de surface qui est de forme annulaire et destiné à être placé autour de la bouteille à traiter, de préférence au niveau de la partie médiane de la bouteille, préférentiellement légèrement plus proche du fond de la bouteille que de son goulot, ledit dispositif étant alimenté par un générateur de micro-ondes.

12. Dispositif générateur de plasma froid de type à cathode creuse, la cathode creuse étant conformée à la forme de la bouteille et le plasma étant alimenté par une polarisation négative continue pulsée et/ou une polarisation radiofréquence.

13. Dispositif selon la revendication 12 **caractérisé par le fait que** la cathode creuse est constituée de deux demi-coques.

14. Dispositif selon la revendication 12 ou 13, **caractérisé par le fait que** la cathode creuse est constituée par le moule d'extrusion-soufflage.
